# EUROPEAN PATENT APPLICATION

(11) **EP 4 818 196 A1**
(43) Date of publication of application: **07.10.2026**
(21) Application number: 24896051.0
(22) Date of filing: 17.10.2024
(51) Int. Cl.: C07K 14/34, C12N 15/31

(54) **USE OF CELL DEATH-RELATED PROTEIN IN PREPARING L-LYSINE**

(30) Priority: 30.11.2023 CN 202311620424
(71) Applicant: Ningxia Eppen Biotech Co. Ltd, Ningxia 750100 (CN)
(72) Inventor: WEI, Aiying, Yinchuan, Ningxia 750100 (CN); MENG, Gang, Yinchuan, Ningxia 750100 (CN); ZHOU, Xiaoqun, Yinchuan, Ningxia 750100 (CN); MA, Fengyong, Yinchuan, Ningxia 750100 (CN); LI, Wengang, Yinchuan, Ningxia 750100 (CN); MA, Wenyou, Yinchuan, Ningxia 750100 (CN); TIAN, Bin, Yinchuan, Ningxia 750100 (CN)
(74) Representative: Winter, Brandl - Partnerschaft mbB
(86) International application number: PCT/CN2024/125414
(87) International publication number: WO 2025/112958

(57) **Abstract**

The present application discloses an application of cell death-related proteins for preparation of L-lysine in the field of biotechnology, and the technical problem to be solved by the present application is how to provide a strain capable of reducing toxic substances or eliminating foam during fermentation to achieve high production of L-lysine, and to solve the technical problem, the application provided by the present application is use of a protein or a substance that regulates expression of a gene encoding the protein or a substance that regulates activity or content of the protein for preparation of L-lysine or improving L-lysine production, wherein the protein is specifically one or more of SEQ ID No.2, SEQ ID No.4, SEQ ID No.7, SEQ ID No.10 and SEQ ID No.12, and the present disclosure proves through experiments that knocking out the gene encoding the aforementioned protein is helpful for improving L-lysine production in the strain.

## Description

### TECHNICAL FIELD

The present application relates to the use of cell death-related proteins in the preparation of L-lysine in the field of biotechnology.

### BACKGROUND

Microbial fermentation is currently the main method for producing lysine, which features low raw material cost, mild reaction conditions, and ease of large-scale production. However, there is still considerable room for improvement in the fermentation performance and conversion rate of lysine-producing strains. During the production of lysine by microbial fermentation, as lysine accumulates, a large amount of toxic substances are generated during the fermentation process, leading to cell autolysis. Moreover, excessive foam is produced during fermentation, necessitating an increased dosage of antifoaming agents, which raises production costs. Furthermore, the excessive foam also reduces the lysine yield of the bacterial cells. Therefore, how to eliminate or alleviate the stress inhibition caused by the aforementioned toxic substances and foam is the key to obtaining high-efficiency lysine-producing strains.

### SUMMARY

The technical problem to be solved by the present application is a strain capable of reducing toxic substances or eliminating foam during fermentation to achieve high-yield production of L-lysine. The technical problems to be solved are not limited to the described technical subject matters, and those skilled in the art can clearly understand other technical subject matters not mentioned herein from the following description.

To solve the above technical problems, the present application provides the following technical solutions.

The present application provides a use, wherein the use comprises D1), D2), D3) or D4).
D1) use of a protein in the preparation of L-lysine or in increasing the yield of L-lysine;
D2) use of a substance that regulates the expression of a gene encoding the protein in the preparation of L-lysine or in increasing the yield of L-lysine;
D3) use of a substance that regulates the activity of the protein in the preparation of L-lysine or in increasing the yield of L-lysine; and
D4) use of a substance that regulates the content of the protein in the preparation of L-lysine or in increasing the yield of L-lysine.

The above protein comprises G1), G2), G3), G4) or G5);
G1) comprising the protein encoded by the NCgl1706 gene and the protein encoded by the NCgl1707 gene;
G2) comprising the protein encoded by the NCgl2777 gene;
G3) comprising the protein encoded by the NCgl1050 gene and the protein encoded by the NCgl1051 gene;
G4) comprising the protein encoded by the NCgl1706 gene, the protein encoded by the NCgl1707 gene, and the protein encoded by the NCgl2777 gene; and
G5) comprising any two or more combinations of G1), G2), G3) or G4).
G5) The combination comprises any one of the following:
   P1) comprising the protein encoded by the NCgl1706 gene, the protein encoded by the NCgl1707 gene, the protein encoded by the NCgl1050 gene, and the protein encoded by the NCgl1051 gene;
   P2) comprising the protein encoded by the NCgl1050 gene, the protein encoded by the NCgl1051 gene, the protein encoded by the NCgl2777 gene, the protein encoded by the NCgl1706 gene, and the protein encoded by the NCgl1707 gene; and
   P3) comprising the protein encoded by the NCgl1050 gene, the protein encoded by the NCgl1051 gene, and the protein encoded by the NCgl2777 gene.

The above protein comprises K1), K2), K3), K4), K5), K6) or K7):
K1) the protein consists of 2 proteins: the protein encoded by the NCgl1706 gene and the protein encoded by the NCgl1707 gene;
K2) the protein is the protein encoded by the NCgl2777 gene;
K3) the protein consists of 2 proteins: the protein encoded by the NCgl1050 gene and the protein encoded by the NCgl1051 gene;
K4) the protein consists of 3 proteins: the protein encoded by the NCgl2777 gene, the protein encoded by the NCgl1706 gene, and the protein encoded by the NCgl1707 gene;
K5) the protein consists of 4 proteins: the protein encoded by the NCgl1050 gene, the protein encoded by the NCgl1051 gene, the protein encoded by the NCgl1706 gene, and the protein encoded by the NCgl1707 gene;
K6) the protein consists of 5 proteins: the protein encoded by the NCgl1050 gene, the protein encoded by the NCgl1051 gene, the protein encoded by the NCgl2777 gene, the protein encoded by the NCgl1706 gene, and the protein encoded by the NCgl1707 gene; and
K7) The protein consists of 3 proteins: the protein encoded by the NCgl1050 gene, the protein encoded by the NCgl1051 gene, and the protein encoded by the NCgl2777 gene.

In the above, the protein encoded by the NCgl1050 gene in A1) comprises A1-1), A1-2) or A1-3);
A1-1) a protein having an amino acid sequence comprising SEQ ID NO: 11;
A1-2) a protein derived from A1-1) or having 80% or more identity with the protein shown in A1-1), obtained by substitution and/or deletion and/or addition of amino acid residues in the amino acid sequence shown in SEQ ID NO: 11, and having the same function as the protein encoded by the NCgl1050 gene; and
A1-3) a fusion protein having the same function, obtained by linking a tag to the N-terminus and/or C-terminus of A1-1) or A1-2).

In the above, the protein encoded by the NCgl1051 gene in A2) comprises A2-1), A2-2) or A2-3);
A2-1) a protein having an amino acid sequence comprising SEQ ID NO: 12;
A2-2) a protein derived from A2-1) or having 80% or more identity with the protein shown in A2-1), obtained by substitution and/or deletion and/or addition of amino acid residues in the amino acid sequence shown in SEQ ID NO: 12, and having the same function as the protein encoded by the NCgl1051 gene; and
A2-3) a fusion protein having the same function, obtained by linking a tag to the N-terminus and/or C-terminus of A2-1) or A2-2).

In the above, the protein encoded by the NCgl2777 gene in A3) comprises A3-1), A3-2) or A3-3);
A3-1) a protein having an amino acid sequence comprising SEQ ID NO: 7;
A3-2) a protein derived from A3-1) or having 80% or more identity with the protein shown in A3-1), obtained by substitution and/or deletion and/or addition of amino acid residues in the amino acid sequence shown in SEQ ID NO: 7, and having the same function as the protein encoded by the NCgl2777 gene; and
A3-3) a fusion protein having the same function, obtained by linking a tag to the N-terminus and/or C-terminus of A3-1) or A3-2).

In the above, the protein encoded by the NCgl1706 gene in A4) comprises A4-1), A4-2) or A4-3);
A4-1) a protein having an amino acid sequence comprising SEQ ID NO: 2;
A4-2) a protein derived from A4-1) or having 80% or more identity with the protein shown in A4-1), obtained by substitution and/or deletion and/or addition of amino acid residues in the amino acid sequence shown in SEQ ID NO: 2, and having the same function as the protein encoded by the NCgl1706 gene; and
A4-3) a fusion protein having the same function, obtained by linking a tag to the N-terminus and/or C-terminus of A4-1) or A4-2).

In the above, the protein encoded by the NCgl1707 gene in A5) comprises A5-1), A5-2) or A5-3);
A5-1) a protein having an amino acid sequence comprising SEQ ID NO: 4;
A5-2) a protein derived from A5-1) or having 80% or more identity with the protein shown in A5-1), obtained by substitution and/or deletion and/or addition of amino acid residues in the amino acid sequence shown in SEQ ID NO: 4, and having the same function as the protein encoded by the NCgl1707 gene; and
A5-3) a fusion protein having the same function, obtained by linking a tag to the N-terminus and/or C-terminus of A5-1) or A5-2).

The above identity refers to amino acid sequence identity. The identity of amino acid sequences can be determined using homology search sites available on the Internet, such as the BLAST webpage on the NCBI homepage. For example, in Advanced BLAST 2.1, using blastp as the program, setting the Expect value to 10, setting all Filters to OFF, using BLOSUM62 as the Matrix, setting the Gap existence cost, Per residue gap cost, and Lambda ratio to 11, 1, and 0.85 (default values), respectively, and performing a search to calculate the identity of a pair of amino acid sequences, the identity value (%) can then be obtained.

The above 80% or more identity may be 80%, 85%, 90%, or 95% or more identity. The above 80% or more identity may be at least 80%, 81%, 82%, 83%, 84%, 85%, 86%, 87%, 88%, 89%, 90%, 91%, 92%, 93%, 94%, 95%, 96%, 97%, 98%, or 99% identity. The above 85% or more identity may be at least 85%, 86%, 87%, 88%, 89%, 90%, 91%, 92%, 93%, 94%, 95%, 96%, 97%, 98%, or 99% identity. The above 90% or more identity may be at least 90%, 91%, 92%, 93%, 94%, 95%, 96%, 97%, 98%, or 99% identity. The above 95% or more identity may be at least 95%, 96%, 97%, 98%, or 99% identity.

In the present application, the "regulation" may be up-regulation or enhancement or increase of the content and/or activity of the aforementioned protein in the cell, or may be down-regulation or inhibition or decrease of the content and/or activity of the aforementioned protein in the cell.

In some specific embodiments of the present application, the regulation of the expression of the gene encoding the protein may specifically be inhibition or reduction or down-regulation of the expression of the encoding gene. The inhibition or reduction or down-regulation of the expression of the encoding gene may be achieved by gene knockout or gene silencing.

The gene knockout refers to the phenomenon of inactivating a specific target gene through homologous recombination. Gene knockout inactivates a specific target gene through changes in the DNA sequence.

The gene silencing refers to the phenomenon of rendering a gene unexpressed or lowly expressed without damaging the original DNA. Gene silencing is premised on not altering the DNA sequence, thereby rendering the gene unexpressed or lowly expressed. Gene silencing can occur at two levels: one is transcriptional gene silencing caused by DNA methylation, heterochromatinization, position effects, etc., and the other is post-transcriptional gene silencing, i.e., inactivation of a gene through specific inhibition of target RNA at the post-transcriptional level, including antisense RNA, co-suppression, quelling, RNA interference (RNAi), and microRNA (miRNA)-mediated translational inhibition.

In the present application, the substance is U1), U2), U3), U4), U5) or U6): U1) regulation at the transcriptional level of the gene; U2) regulation at the post-transcriptional level of the gene (i.e., regulation of the splicing or processing of the primary transcript of the gene); U3) regulation of RNA transport of the gene (i.e., regulation of the transport of mRNA of the gene from the nucleus to the cytoplasm); U4) regulation of translation of the gene; U5) regulation of mRNA degradation of the gene; U6) regulation at the post-translational level of the gene (i.e., regulation of the activity of the protein translated from the gene).

When the regulation is inhibition or reduction or down-regulation, the substance may be an agent for knocking out the gene encoding the protein, such as an agent for knocking out the gene encoding the protein through homologous recombination, or an agent for knocking out the gene encoding the protein through the CRISPR-Cas system.

The agent includes B1), B2), B3), B4) or B5):
B1) a nucleic acid molecule that inhibits or reduces or down-regulates the expression of the gene encoding the protein, or a nucleic acid molecule that inhibits or reduces or down-regulates the activity or content of the protein; the nucleic acid molecule includes a DNA molecule or an RNA molecule;
B2) a gene encoding the RNA molecule of B1);
B3) an expression cassette containing the gene of B2);
B4) a recombinant vector containing the gene of B2), or a recombinant vector containing the expression cassette of B3); and
B5) a recombinant microorganism containing the gene of B2), or a recombinant microorganism containing the expression cassette of B3), or a recombinant microorganism containing the recombinant vector of B4).

In the above, the DNA molecule of B1) includes a nucleic acid molecule for knocking out the gene encoding the protein in a cell through homologous recombination (such as circular DNA (e.g., a vector) or linear DNA).

The nucleotide sequence of the NCgl1050 gene contains the sequence of SEQ ID NO: 9, the nucleotide sequence of the NCgl1051 gene contains the sequence of SEQ ID NO: 11 (540 bp), the nucleotide sequence of the NCgl2777 gene contains the sequence of SEQ ID NO: 6 (1974 bp), the nucleotide sequence of the NCgl1706 gene contains the sequence of SEQ ID NO: 1 (1524 bp), and the nucleotide sequence of the NCgl1707 gene contains the sequence of SEQ ID NO: 3 (672 bp).

The present application also provides a recombinant strain, wherein the recombinant strain does not contain or lacks the aforementioned protein.

Further, the recombinant strain does not contain or lacks the gene encoding the aforementioned protein.

Further, the recombinant strain comprises a bacterium. The recombinant strain may specifically be a bacterium.

The recombinant strain may be obtained by knocking out or silencing the gene encoding the protein in a host strain, wherein the host strain contains the gene encoding the protein.

Further, the recombinant strain comprises Corynebacterium glutamicum. The recombinant strain may specifically be Corynebacterium glutamicum.

The use of biological materials related to the aforementioned protein in the preparation of or improvement in the production of glutamic acid, lysine, valine, glycine, alanine, leucine, isoleucine, methionine, proline, tryptophan, serine, tyrosine, cysteine, phenylalanine, asparagine, glutamine, threonine, aspartic acid, arginine, histidine, shikimic acid, protocatechuic acid, succinic acid, α-ketoglutaric acid, citric acid, ornithine, or citrulline, preferably in the preparation of L-lysine or in the improvement of L-lysine yield, also falls within the scope of protection of the present application.

The biological material includes:
B1) a nucleic acid molecule that inhibits or reduces or down-regulates the expression of the gene encoding the protein, or a nucleic acid molecule that inhibits or reduces or down-regulates the activity or content of the protein; the nucleic acid molecule includes a DNA molecule or an RNA molecule;
B2) a gene encoding the RNA molecule of B1);
B3) an expression cassette containing the gene of B2);
B4) a recombinant vector containing the gene of B2), or a recombinant vector containing the expression cassette of B3); and
B5) a recombinant microorganism containing the gene of B2), or a recombinant microorganism containing the expression cassette of B3), or a recombinant microorganism containing the recombinant vector of B4).

The expression cassette in the above biological material refers to a DNA capable of expressing the aforementioned protein in a host cell. The expression cassette may further include a single-stranded or double-stranded nucleic acid molecule comprising all regulatory sequences necessary for the expression of any one of the aforementioned proteins by the nucleic acid molecule. The regulatory sequences are capable of directing the expression of any one of the aforementioned proteins from the coding sequence in a suitable host cell under compatible conditions. The regulatory sequences include, but are not limited to, a leader sequence, a polyadenylation sequence, a propeptide sequence, a promoter, a signal sequence, and a transcription terminator. At a minimum, the regulatory sequences shall include a promoter as well as transcription and translation termination signals. In order to introduce specific restriction enzyme sites into the vector for ligating the regulatory sequences to the coding region of the nucleic acid sequence encoding the protein, regulatory sequences with adapters may be provided. The regulatory sequences may be suitable promoter sequences, i.e., nucleic acid sequences recognizable by the host cell expressing the nucleic acid sequence. The promoter sequence contains transcription regulatory sequences that mediate protein expression. The promoter may be any nucleic acid sequence having transcriptional activity in the selected host cell, including mutated, truncated, and hybrid promoters, and may be derived from genes encoding extracellular or intracellular proteins homologous or heterologous to the host cell. The regulatory sequences may also be suitable transcription termination sequences, i.e., a sequence recognizable by the host cell to terminate transcription. The termination sequence is operably linked to the 3' end of the nucleic acid sequence encoding the protein. Any terminator functional in the selected host cell may be used in the present application. The regulatory sequences may also be suitable leader sequences, i.e., untranslated regions of mRNA that are important for translation in the host cell. The leader sequence is operably linked to the 5' end of the nucleic acid sequence encoding the protein. Any leader sequence functional in the selected host cell may be used in the present application. The regulatory sequences may also be a signal peptide coding region, which encodes an amino acid sequence linked to the amino terminus of the protein and is capable of directing the encoded protein into the cellular secretion pathway. Any signal peptide coding region capable of directing the expressed protein into the secretion pathway of the host cell used may be used in the present application. It may also be necessary to add regulatory sequences capable of modulating protein expression according to the growth conditions of the host cell. Examples of regulatory systems are those capable of responding to chemical or physical stimuli (including in the presence of regulatory compounds) to turn gene expression on or off. Other examples of regulatory sequences are those capable of enabling gene amplification. In these examples, the nucleic acid sequence encoding the protein shall be operably linked to the regulatory sequences.

The recombinant vector may include a nucleic acid molecule encoding the aforementioned protein, a promoter, and transcription and translation termination signals. When preparing the recombinant vector, the nucleic acid molecule encoding the aforementioned protein may be positioned in the vector for operable linkage with appropriate expression regulatory sequences. The recombinant vector may be any vector convenient for recombinant DNA manipulation and expression of the nucleic acid sequence (e.g., a plasmid or a virus). The choice of vector typically depends on the compatibility of the vector with the host cell into which it is to be introduced. The vector may be a linear or closed-circular plasmid. The vector may be an autonomously replicating vector (i.e., a complete structure existing extrachromosomally that can replicate independently of the chromosome), such as a plasmid, an extrachromosomal element, a minichromosome, or an artificial chromosome. The vector may contain any mechanism that ensures self-replication. Alternatively, the vector is one that, when introduced into a host cell, integrates into the genome and replicates together with the chromosome into which it is integrated. Furthermore, a single vector or plasmid, or two or more vectors or plasmids collectively containing all the DNA to be introduced into the host cell genome, or a transposon may be used. The vector contains one or more selection markers for facilitating the selection of transformed cells. A selection marker is a gene whose product confers resistance to a biocide or virus, resistance to a heavy metal, or confers prototrophy on an auxotroph, etc. Examples of bacterial selection markers include the dal gene of Bacillus subtilis or Bacillus licheniformis, or resistance markers for antibiotics such as ampicillin, kanamycin, chloramphenicol, or tetracycline. The vector contains elements that enable stable integration of the vector into the host cell genome, or ensure autonomous replication of the vector in the cell independent of the cell genome. In the case of autonomous replication, the vector may also contain an origin of replication that enables the vector to replicate autonomously in the target host cell. The origin of replication may carry mutations that render it temperature-sensitive in the host cell (see, for example, Ehrlich, 1978, Proceedings of the National Academy of Sciences 75: 1433). More than one copy of the nucleic acid molecule of the present application encoding any one of the aforementioned proteins may be inserted into the host cell to increase the yield of the gene product. The copy number of the nucleic acid molecule may be increased by inserting at least one additional copy of the nucleic acid molecule into the host cell genome, or by inserting an amplifiable selection marker together with the nucleic acid molecule, and then culturing the cells in the presence of a suitable selection agent to select for cells containing the amplified copy of the selection marker gene, and thus containing the additional copy of the nucleic acid molecule. The operations for ligating the above elements to construct the recombinant expression vector of the present application are well known to those skilled in the art (see, for example, Sambrook et al., Molecular Cloning: A Laboratory Manual, Second Edition, Cold Spring Harbor Laboratory Press, Cold Spring Harbor, New York, 1989).

The term "operably linked" is defined herein as a conformation in which a regulatory sequence is positioned at an appropriate location relative to the coding sequence of a DNA sequence, such that the regulatory sequence directs the expression of the protein.

The present application also provides a method for preparing a recombinant microorganism, comprising the following steps: down-regulating, reducing, or inhibiting the expression level of a gene in a target microorganism, or/and down-regulating, reducing, or inhibiting the expression level or activity of the protein encoded by the gene in the target microorganism, to obtain a recombinant microorganism; the gene comprises the aforementioned NCgl1706 gene, NCgl1707 gene, NCgl2777 gene, NCgl1050 gene, and/or NCgl1051 gene.

Specifically, the gene may be the NCgl1706 gene, NCgl1707 gene, NCgl2777 gene, NCgl1050 gene, and NCgl1051 gene; the gene may be the NCgl2777 gene, the NCgl1706 gene, and the NCgl1707 gene; the gene may also be the NCgl1050 gene, the NCgl1051 gene, and the NCgl2777 gene; the gene may also be the NCgl1050 gene, the NCgl1051 gene, the NCgl1706 gene, and the NCgl1707 gene; the gene may be the NCgl1706 gene and the NCgl1707 gene; the gene may be the NCgl2777 gene; the gene may be the NCgl1050 gene and the NCgl1051 gene.

Further, the target microorganism in the above method comprises Corynebacterium glutamicum. The target microorganism may specifically be Corynebacterium glutamicum. The improvement in L-lysine yield includes improving the L-lysine yield of the bacterium.

The present application also provides a whole-cell catalyst, wherein the whole-cell catalyst comprises the aforementioned recombinant strain or the aforementioned biological material. The present application also provides a method for preparing L-lysine, comprising fermenting and preparing L-lysine using the aforementioned recombinant strain, biological material, or whole-cell catalyst.

### Beneficial Effects

The present application experimentally demonstrates that, compared to the target microorganism Corynebacterium glutamicum YP097158, the L-lysine yield is significantly improved in the recombinant Corynebacterium glutamicum YPL-ΔNCgl1706-NCgl1707-ΔNCgl2777-ΔNCgl1050-NCgl1051 with the NCgl1050 gene, NCgl1051 gene, NCgl2777 gene, NCgl1706 gene, and NCgl1707 gene knocked out, the recombinant Corynebacterium glutamicum YPL-ΔNCgl2777-ΔNCgl1050-NCgl1051 with the NCgl1050 gene, NCgl1051 gene, and NCgl2777 gene knocked out, the recombinant Corynebacterium glutamicum YPL-ΔNCgl1706-NCgl1707-ΔNCgl1050-NCgl1051 with the NCgl1050 gene, NCgl1051 gene, NCgl1706 gene, and NCgl1707 gene knocked out, the recombinant Corynebacterium glutamicum YPL-ΔNCgl1050-NCgl1051 with the NCgl1050 gene and NCgl1051 gene knocked out, the recombinant Corynebacterium glutamicum YPL-ΔNCgl1706-NCgl1707-ΔNCgl2777 with the NCgl2777 gene, NCgl1706 gene, and NCgl1707 gene knocked out, the recombinant Corynebacterium glutamicum YPL-ΔNCgl2777 with the NCgl2777 gene knocked out, and the recombinant Corynebacterium glutamicum YPL-ΔNCgl1706-NCgl1707 with the NCgl1706 gene and NCgl1707 gene knocked out.

### Deposit Information

Strain name: Corynebacterium glutamicum
Latin name: Corynebacterium glutamicum
Strain number: YP097158
Depositary: China General Microbiological Culture Collection Center
Abbreviation of depositary: CGMCC
Address: No. 3, Yard 1, Beichen West Road, Chaoyang District, Beijing, China
Date of deposit: August 16, 2016
Accession number: CGMCC No. 12856.

### BRIEF DESCRIPTION OF THE DRAWINGS

FIG. 1 shows the RT-qPCR detection results of NCgl1706-NCgl1707.
FIG. 2 shows the RT-qPCR detection results of NCg12777.
FIG. 3 shows the RT-qPCR detection results of NCgl1050-NCgl1051.
FIG. 4 shows the RT-qPCR detection results of NCg11706-NCg11707-NCg12777.
FIG. 5 shows the RT-qPCR detection results of NCg11706-NCg11707-NCg11050-NCg11051.
FIG. 6 shows the RT-qPCR detection results of NCgl2777-NCgl1050-NCgl1051.
FIG. 7 shows the RT-qPCR detection results of NCgl1706-NCgl1707-NCgl2777-NCgl1050-NCgl1051.

### DETAILED DESCRIPTION

The present application is further described in detail below in conjunction with specific embodiments. The examples provided are merely for illustrating the present application, and not for limiting the scope of the present application. The examples provided below may serve as a guide for those skilled in the art to make further improvements, and do not constitute any limitation on the present application in any way.

The experimental methods in the following examples are conventional methods unless otherwise specified, and are carried out according to the techniques or conditions described in the literature in the art or according to the product instructions. The materials, reagents, etc. used in the following examples are commercially available unless otherwise specified.

The present application is further described in detail below in conjunction with specific embodiments. The examples provided are merely for illustrating the present application, and not for limiting the scope of the present application. The examples provided below may serve as a guide for those skilled in the art to make further improvements, and do not constitute any limitation on the present application in any way.

The experimental methods in the following examples are conventional methods unless otherwise specified, and are carried out according to the techniques or conditions described in the literature in the art or according to the product instructions. The materials, reagents, etc. used in the following examples are commercially available unless otherwise specified.

### Definitions:

Unless the content explicitly indicates otherwise, as used in this specification and the appended claims, the singular forms "a", "an", and "one" include plural referents.

As used herein, the term "nucleotide" refers to a monomeric unit of a polynucleotide, consisting of a heterocyclic base, a sugar, and one or more phosphate groups. Naturally occurring bases (guanine (G), adenine (A), cytosine (C), thymine (T), and uracil (U)) are typically derivatives of purines or pyrimidines, but it should be understood that naturally and non-naturally occurring base analogs are also included. Naturally occurring sugars are pentoses (five-carbon sugars), deoxyribose (which forms DNA), or ribose (which forms RNA), but it should be understood that naturally and non-naturally occurring sugar analogs are also included. Nucleic acids are typically linked via phosphodiester bonds to form nucleic acids or polynucleotides, and may also be linked via other bonds known in the art, such as phosphorothioate bonds.

As used herein, the terms "polypeptide" and "protein" are used interchangeably and refer to a polymer of amino acid residues.

As used herein, the term "vector" refers to a nucleic acid molecule capable of delivering another nucleic acid (i.e., a polynucleotide sequence) linked thereto.

As used herein, the term "fermentation" broadly refers to the conversion of organic materials by host cells into a target substance, for example, by culturing a recombinant host cell in a medium comprising a carbon source such that the recombinant host cell converts the carbon source into lysine.

As used herein, the term "L-lysine" refers to L-2,6-diaminocaproic acid (L-Lysine).

The wild-type Corynebacterium glutamicum ATCC13032 strain: a product of the American Type Culture Collection (ATCC).

The Corynebacterium glutamicum YP097158 strain used in the following examples is described in the Chinese patent document with authorization number "CN110607313B" and entitled "A recombinant strain with high L-lysine production and its construction method and application". The strain number is YP097158, which is deposited with the China General Microbiological Culture Collection Center (CGMCC) on August 16, 2016, under deposit number CGMCC No. 12856. Hereinafter referred to as Corynebacterium glutamicum CGMCC No. YP097158.

In the following examples, data are processed using GraphPad Prism 8 statistical software. Experimental results are expressed as mean ± standard deviation. One-way ANOVA is used for statistical analysis, and P < 0.05 (*) indicates a statistically significant difference.

All quantitative experiments in the following examples are performed with three replicates, and the results are expressed as mean values. The 2^{-ΔΔCt} method is used to analyze the qRT-PCR results and calculate the relative gene expression levels.

### Example 1: Construction of an engineered strain with deletion of the NCgl1706-NCgl1707 genes on the genome

Based on the Corynebacterium glutamicum ATCC13032 sequence published by NCBI, the pK18mobsacB plasmid is used to knock out the NCgl1706-NCgl1707 genes in the genome of Corynebacterium glutamicum YP097158 (derived from Corynebacterium glutamicum ATCC13032) (sequencing confirmed that the YP097158 strain retains intact NCgl1706-NCgl1707 genes on the chromosome), in order to further study the effects of these genes on L-lysine synthesis.

### I. Construction of the knockout plasmid

Based on the genome sequence of Corynebacterium glutamicum ATCC13032 published by NCBI, two pairs of primers are synthesized to amplify the flanking fragments of the NCgl1706-NCgl1707 genes, which served as the upstream and downstream homologous arm fragments. The primers are designed as follows (synthesized by Shanghai Invitrogen Co., Ltd.): 
P1:5'-CAGTGCCAAGCTTGCATGCCTGCAGGTCGACTCTAGAATCCAAGCGACCACA
AG-3' (SEQ ID NO: 14);
P2: 5'-CTATTTTCAGGAACATTTACGTGTCGTGAGCGATAAAAAACAC-3' (SEQ ID NO: 15);
P3: 5'-GTGTTTTTTATCGCTCACGACACGTAAATGTTCCTGAAAATAG-3' (SEQ ID NO: 16);
P4:5'-CAGCTATGACCATGATTACGAATTCGAGCTCGGTACCCCTCGTGCGATT-3' (SEQ ID NO: 17).

In the above primers, the underlined nucleotide sequences are homologous sequences on pK18, which are used for integrating the DNA fragment (upstream homologous arm - downstream homologous arm) into the pK18mobsacB plasmid (Addgene) via homologous recombination. P2 and P3 are used for linking the upstream homologous arm and the downstream homologous arm via homologous recombination.

Construction method: Using the Corynebacterium glutamicum ATCC13032 genome as a template, PCR amplification is performed using primers P1/P2 and P3/P4, respectively, with the high-fidelity DNA polymerase KAPA HiFi HotStart (purchased from Shanghai Huayasi Chuang Biotechnology Co., Ltd., KK2601), to obtain a 797 bp upstream homologous arm fragment for knocking out the NCgl1706-NCgl1707 genes (the sequence is as shown in bases 1-797 of SEQ ID NO: 5) and a 693 bp downstream homologous arm fragment (the sequence is as shown in bases 798-1490 of SEQ ID NO: 5). The amplified products are subjected to electrophoresis and purified using a column-type DNA gel recovery kit. The two recovered DNA fragments and the pK18mobsacB plasmid (purchased from Addgene) digested with XbaI/BamHI and purified are ligated at 50°C for 30 min using NEBuilder enzyme (purchased from NEB). Monoclonal colonies grown after transformation of the ligation products are identified by PCR using primers M13F (5'-TGTAAAACGACGGCCAGT-3', SEQ ID NO: 18) / M13R (5'-CAGGAAACAGCTATGACC-3', SEQ ID NO: 19) to obtain the positive knockout vector pK18-ΔNCgl1706-NCgl1707, which contains a 1490 bp homologous DNA fragment (sequence: SEQ ID NO: 5) for knocking out the NCgl1706-NCgl1707 genes. The plasmid contains a kanamycin resistance gene as a selection marker, and the plasmid is sent for sequencing.

PCR amplification system: 5×HiFi with Mg2+ Buffer 10 µL, dNTP Mixture (10 mM) 1.5 µL, primers (10 µM) 1.6 µL each, KAPA HiFi HotStart (1 U/µL) 0.5 µL, ddH₂O added to a total volume of 50 µL. PCR amplification program: 95°C pre-denaturation for 5 min, (98°C denaturation for 20 s; 60°C annealing for 15 s; 72°C extension for 30 s; 30 cycles), 72°C over-extension for 5 min.

### II. Construction of the knockout strain

The sequenced knockout plasmid pK18-ΔNCgl1706-NCgl1707 is electrotransformed into Corynebacterium glutamicum YP097158. The bacteria are cultured according to the medium composition and culture conditions shown in Table 1. Single colonies generated from the culture are identified by PCR using primers P1/P4: strains that could simultaneously amplify bands of 1490 bp and 3931 bp are positive strains, while strains that only amplified a 3931 bp band are the original strain. The positive strains are cultured on 15% sucrose solid medium (shown in Table 1) for 30 h, and then single clones are picked and screened on solid medium plates containing kanamycin and without kanamycin. Single clone strains that grow on the medium without kanamycin but do not grow on the medium containing kanamycin are further identified by PCR using primers P1/P4. Strains that could amplify a 1490 bp (SEQ ID NO: 5) band are positive strains with the coding region of the NCgl1706-NCgl1707 genes knocked out. The gene fragment of the positive strain (with the coding region of NCgl1706-NCgl1707 genes knocked out) is amplified again using primers P1/P4 and sequenced. The strain with correct sequencing is designated YPL-ΔNCgl1706-NCgl1707.

The recombinant strain YPL-ΔNCgl1706-NCgl1707 is a recombinant Corynebacterium glutamicum obtained by replacing bases 1881670 to 1885600 of the ATCC13032 genome (GenBank: NC_03450, 22-DEC-2014) with SEQ ID NO: 5, while keeping other nucleotide sequences unchanged. Specifically, the coding region of the NCgl1706 gene (nucleotide sequence: SEQ ID NO: 1, encoding the amino acid sequence shown in SEQ ID NO: 2, i.e., bases 1882467 to 1883990 of the ATCC13032 genome (GenBank: NC_03450, 22-DEC-2014)) and the coding region of the NCgl1707 gene (sequence: SEQ ID NO: 3, encoding the amino acid sequence shown in SEQ ID NO: 4, i.e., bases 1884217 to 1884888 of the ATCC13032 genome (GenBank: NC_03450, 22-DEC-2014)) in the genome of Corynebacterium glutamicum YP097158 are knocked out, while other nucleotides in the genome of Corynebacterium glutamicum YP097158 are kept unchanged.

**Table 1: Medium composition and culture conditions**

| Component | Formula | Component | Formula |
|---|---|---|---|
| Glucose | 5 g/L | NaCl | 2.5 g/L |
| Soybean meal extract | 15g/L | KH₂PO₄ | 1 g/L |
| Yeast powder | 10 g/L | Agar | 18 g/L |
| Urea | 3g/L | pH | 7 |

| | | | |
|---|---|---|---|
| Note: The culture temperature is 32°C, and the culture time is 30 h. | | | |

### III. RT-qPCR detection of the recombinant strain

The recombinant strain YPL-ΔNCgl1706-NCgl1707 and Corynebacterium glutamicum YP097158 are each cultured for 24 h, and then the cells are collected by centrifugation at 4°C. RNA is extracted using an RNA extraction kit (purchased from Takara, Code No.: 9108). RNA is reverse transcribed into cDNA using a Premix-type reverse transcription kit (purchased from Takara, Code No.: RR036Q). The transcription levels of the NCgl1706 and NCgl1707 genes are detected using a qPCR kit (purchased from Takara, Code No.: RR42LR). The 16S rRNA gene is selected as the internal reference gene. The primers are designed as follows:
NCgl1706-F: 5'-GACCGTGACCATCTCTACATC-3' (SEQ ID NO: 20);
NCgl1706-R: 5'-TTCATTGTGTGACCACAC-3' (SEQ ID NO: 21);
NCgl1707-F: 5'-TGTATCCGTAGTAATCCTGGC-3' (SEQ ID NO: 22);
NCgl1707-R: 5'-GGGACGATGAATCCAAATG-3' (SEQ ID NO: 23).

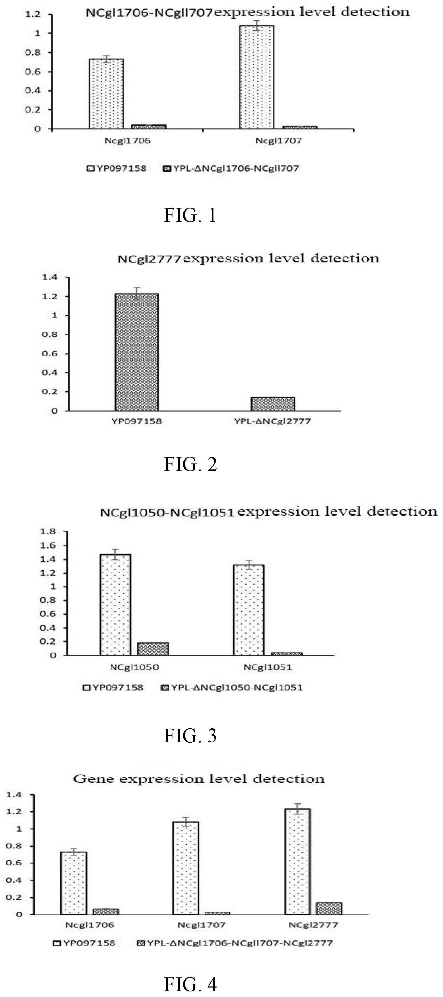

As can be seen from FIG. 1, compared with Corynebacterium glutamicum YP097158, the expression levels of the NCgl1706 and NCgl1707 genes in the recombinant strain YPL-ΔNCg11706-NCg11707 are significantly decreased, confirming successful knockout.

### Example 2: Construction of an engineered strain with deletion of the NCgl2777 gene on the genome

Based on the Corynebacterium glutamicum ATCC13032 sequence published by NCBI, the pK18mobsacB plasmid is used to knock out the NCgl2777 gene in the genome of the L-lysine-producing strain YP097158 (derived from Corynebacterium glutamicum ATCC13032) (sequencing confirmed that the YP097158 strain retains an intact NCgl2777 gene on the chromosome), in order to further study the effects of these genes on L-lysine synthesis.

### I. Construction of the knockout plasmid

Based on the genome sequence of Corynebacterium glutamicum ATCC13032 published by NCBI, two pairs of primers are synthesized to amplify the flanking fragments of the NCgl2777 gene, which served as the upstream and downstream homologous arm fragments. The primers are designed as follows (synthesized by Shanghai Invitrogen Co., Ltd.): 
P5:5'-CAGTGCCAAGCTTGCATGCCTGCAGGTCGACTCTAGACAGCAATAACGGTGAT
GG-3' (SEQ ID NO: 24);
P6: 5'-CAAAATGAGAAGGAAAACTTCCAATGAGAAGTTGATCAAC-3' (SEQ ID NO: 25);
P7: 5'-GTTGATCAACTTCTCATTGGAAGTTTTCCTTCTCATTTTG-3' (SEQ ID NO: 26);

P8:5'-CAGCTATGACCATGATTACGAATTCGAGCTCGGTACCCTCGGATTTGGAGATGT
CG-3' (SEQ ID NO: 27).

In the above primers, the underlined nucleotide sequences are homologous sequences on pK18, which are used for integrating the DNA fragment (upstream homologous arm - downstream homologous arm) into the pK18mobsacB plasmid (Addgene) via homologous recombination. P6 and P7 are used for linking the upstream homologous arm and the downstream homologous arm via homologous recombination.

Construction method: using the Corynebacterium glutamicum ATCC13032 genome as a template, PCR amplification is performed using primers P5/P6 and P7/P8, respectively, with the high-fidelity DNA polymerase KAPA HiFi HotStart (purchased from Shanghai Huayasi Chuang Biotechnology Co., Ltd., KK2601), to obtain a 788 bp upstream homologous arm fragment for knocking out the NCgl2777 gene (nucleotide sequence as shown in bases 1-788 of SEQ ID NO: 8) and a 692 bp downstream homologous arm fragment (nucleotide sequence as shown in bases 789-1480 of SEQ ID NO: 8). The amplified products are subjected to electrophoresis and purified using a column-type DNA gel recovery kit. The two recovered DNA fragments and the pK18mobsacB plasmid (purchased from Addgene) digested with XbaI/BamHI and purified are ligated at 50°C for 30 min using NEBuilder enzyme (purchased from NEB). Monoclonal colonies grown after transformation of the ligation products are identified by PCR using primers M13F/M13R to obtain the positive knockout vector pK18-ΔNCgl2777, which contains a 1480 bp homologous DNA fragment (SEQ ID NO: 8) for knocking out the NCgl2777 gene. The plasmid contains a kanamycin resistance gene as a selection marker, and the plasmid is sent for sequencing.

PCR amplification system: 5×HiFi with Mg²⁺ Buffer 10 µL, dNTP Mixture (10 mM) 1.5 µL, primers (10 µM) 1.6 µL each, KAPA HiFi HotStart (1 U/µL) 0.5 µL, ddH₂O added to a total volume of 50 µL. PCR amplification program: 95°C pre-denaturation for 5 min, (98°C denaturation for 20 s; 60°C annealing for 15 s; 72°C extension for 30 s; 30 cycles), 72°C over-extension for 5 min.

### II. Construction of the knockout strain

The sequenced knockout plasmid pK18-ΔNCgl2777 is electrotransformed into Corynebacterium glutamicum YP097158. The bacteria are cultured according to the medium composition and culture conditions shown in Table 1. Single colonies generated from the culture are identified by PCR using primers P5/P8: strains that could simultaneously amplify bands of 1480 bp and 3201 bp are positive strains, while strains that only amplified a 3201 bp band are the original strain. The positive strains are cultured on 15% sucrose solid medium (shown in Table 1) for 30 h, and then single clones are picked and screened on solid medium plates containing kanamycin and without kanamycin. Single clone strains that grow on the medium without kanamycin but do not grow on the medium containing kanamycin are further identified by PCR using primers P5/P8. Strains that could amplify a 1480 bp (SEQ ID NO: 8) band are positive strains with the coding region of the NCgl2777 gene knocked out. The gene fragment of the positive strain (with the coding region of the NCgl2777 gene knocked out) is amplified again using primers P5/P8 and sequenced. The strain with correct sequencing is designated YPL-ΔNCgl2777.

The recombinant strain YPL-ΔNCgl2777 is a recombinant Corynebacterium glutamicum obtained by replacing bases 3071113 to 3074313 of the ATCC13032 genome (GenBank: NC_03450, 22-DEC-2014) with SEQ ID NO: 8, while keeping other nucleotide sequences unchanged. Specifically, the coding region of the NCgl2777 gene (sequence as shown in SEQ ID NO: 6, encoding the amino acid sequence shown in SEQ ID NO: 7, i.e., bases 3071901 to 3073621 of the ATCC13032 genome (GenBank: NC_03450, 22-DEC-2014)) in the genome of Corynebacterium glutamicum YP097158 is knocked out, while other nucleotides in the genome of Corynebacterium glutamicum YP097158 are kept unchanged.

### III. RT-qPCR detection of the recombinant strain

The recombinant strain YPL-ΔNCgl2777 and Corynebacterium glutamicum YP097158 are each cultured for 24 h, and then the cells are collected by centrifugation at 4°C. RNA is extracted using an RNA extraction kit (purchased from Takara, Code No.: 9108). RNA is reverse transcribed into cDNA using a Premix-type reverse transcription kit (purchased from Takara, Code No.: RR036Q). The transcription level of the NCgl2777 gene is detected using a qPCR kit (purchased from Takara, Code No.: RR42LR). The 16S rRNA gene is selected as the internal reference gene. The primers are designed as follows:
NCgl2777-F: 5'-CCTGAAATCTGGGCACTTG-3' (SEQ ID NO: 28);
NCgl2777-R: 5'-TTAGAACGGAAACCCTTGTCC-3' (SEQ ID NO: 29).

As can be seen from FIG. 2, compared with Corynebacterium glutamicum YP097158, the expression level of the NCgl2777 gene in the recombinant strain YPL-ΔNCgl2777 is significantly decreased, confirming successful knockout of the NCgl2777 gene.

### Example 3: Construction of an engineered strain with deletion of the NCgl1050-NCgl1051 genes on the genome

Based on the Corynebacterium glutamicum ATCC13032 sequence published by NCBI, the pK18mobsacB plasmid is used to knock out the NCgl1050-NCgl1051 genes in the genome of the L-lysine-producing strain YP097158 (derived from Corynebacterium glutamicum ATCC13032) (sequencing confirmed that the YP097158 strain retains intact NCgl1050-NCgl1051 genes on the chromosome), in order to further study the effects of these genes on L-lysine synthesis.

### I. Construction of the knockout plasmid

Based on the genome sequence of Corynebacterium glutamicum ATCC13032 published by NCBI, two pairs of primers are synthesized to amplify the flanking fragments of the NCgl1050-NCgl1051 genes, which served as the upstream and downstream homologous arm fragments. The primers are designed as follows (synthesized by Shanghai Invitrogen Co., Ltd.): 
P9:5'-CAGTGCCAAGCTTGCATGCCTGCAGGTCGACTCTAGAACTTGCCTGATTGGGA
AG-3' (SEQ ID NO: 30);
P10: 5'-GATGTGAGGTAAGAAAACAGATGCGAAACCGGCGCCAAC-3' (SEQ ID NO: 31);
P11: 5'-GTTGGCGCCGGTTTCGCATCTGTTTTCTTACCTCACATC-3' (SEQ ID NO: 32);

P12:5'-CAGCTATGACCATGATTACGAATTCGAGCTCGGTACCCGTGAATCTTTAGGGA
AACCCAG-3' (SEQ ID NO: 33).

In the above primers, the underlined nucleotide sequences are homologous sequences on pK18, which are used for integrating the DNA fragment (upstream homologous arm - downstream homologous arm) into the pK18mobsacB plasmid (Addgene) via homologous recombination. P10 and P11 are used for linking the upstream homologous arm and the downstream homologous arm via homologous recombination.

Construction method: Using the Corynebacterium glutamicum ATCC13032 genome as a template, PCR amplification is performed using primers P9/P10 and P11/P12, respectively, with the high-fidelity DNA polymerase KAPA HiFi HotStart (purchased from Shanghai Huayasi Chuang Biotechnology Co., Ltd., KK2601), to obtain an 834 bp upstream homologous arm fragment for knocking out the NCgl1050-NCgl1051 genes (sequence as shown in bases 1-834 of SEQ ID NO: 13) and an 882 bp downstream homologous arm fragment (sequence as shown in bases 835-1716 of SEQ ID NO: 13). The amplified products are subjected to electrophoresis and purified using a column-type DNA gel recovery kit. The two recovered DNA fragments and the pK18mobsacB plasmid (purchased from Addgene) digested with XbaI/BamHI and purified are ligated at 50°C for 30 min using NEBuilder enzyme (purchased from NEB). Monoclonal colonies grown after transformation of the ligation products are identified by PCR using primers M13F/M13R to obtain the positive knockout vector pK18-ΔNCgl1050-NCgl1051, which contains a 1716 bp homologous DNA fragment (SEQ ID NO: 13) for knocking out the NCgl1050-NCgl1051 genes. The plasmid contains a kanamycin resistance gene as a selection marker, and the plasmid is sent for sequencing.

PCR amplification system: 5×HiFi with Mg²⁺ Buffer 10 µL, dNTP Mixture (10 mM) 1.5 µL, primers (10 µM) 1.6 µL each, KAPA HiFi HotStart (1 U/µL) 0.5 µL, ddH₂O added to a total volume of 50 µL.

PCR amplification program: 95°C pre-denaturation for 5 min, (98°C denaturation for 20 s; 60°C annealing for 15 s; 72°C extension for 30 s; 30 cycles), 72°C over-extension for 5 min.

### II. Construction of the knockout strain

The sequenced knockout plasmid pK18-ΔNCgl1050-NCgl1051 is electrotransformed into Corynebacterium glutamicum YP097158. The bacteria are cultured according to the medium composition and culture conditions shown in Table 1. Single colonies generated from the culture are identified by PCR using primers P9/P12: strains that could simultaneously amplify bands of 1716 bp and 3391 bp are positive strains, while strains that only amplified a 3391 bp band are the original strain. The positive strains are cultured on 15% sucrose solid medium (shown in Table 1) for 30 h, and then single clones are picked and screened on solid medium plates containing kanamycin and without kanamycin. Single clone strains that grow on the medium without kanamycin but do not grow on the medium containing kanamycin are further identified by PCR using primers P9/P12. Strains that could amplify a 1716 bp (SEQ ID NO: 13) band are positive strains with the coding regions of the NCgl1050-NCgl1051 genes knocked out. The gene fragment of the positive strain (with the coding regions of the NCgl1050-NCgl1051 genes knocked out) is amplified again using primers P9/P12 and sequenced. The strain with correct sequencing is designated YPL-ΔNCgl1050-NCgl1051.

The recombinant strain YPL-ΔNCgl1050-NCgl1051 is a recombinant Corynebacterium glutamicum obtained by replacing bases 1140507 to 1143897 of the ATCC13032 genome (GenBank: NC_03450, 22-DEC-2014) with SEQ ID NO: 13, while keeping other nucleotide sequences unchanged. Specifically, the coding region of the NCgl1050 gene (nucleotide sequence: SEQ ID NO: 9, encoding the amino acid sequence shown in SEQ ID NO: 10, i.e., bases 1141341 to 1142475 of the ATCC13032 genome (GenBank: NC_03450, 22-DEC-2014)) and the coding region of the NCgl1051 gene (nucleotide sequence: SEQ ID NO: 11, encoding the amino acid sequence shown in SEQ ID NO: 12, i.e., bases 1142476 to 1143015 of the ATCC13032 genome (GenBank: NC_03450, 22-DEC-2014)) in the genome of Corynebacterium glutamicum YP097158 are knocked out, while other nucleotides in the genome of Corynebacterium glutamicum YP097158 are kept unchanged.

### III. RT-qPCR detection of the recombinant strain

The recombinant strain YPL-ΔNCgl1050-NCgl1051 and Corynebacterium glutamicum YP097158 are each cultured for 24 h, and then the cells are collected by centrifugation at 4°C. RNA is extracted using an RNA extraction kit (purchased from Takara, Code No.: 9108). RNA is reverse transcribed into cDNA using a Premix-type reverse transcription kit (purchased from Takara, Code No.: RR036Q). The transcription levels of the NCgl1050 and NCgl1051 genes are detected using a qPCR kit (purchased from Takara, Code No.: RR42LR). The 16S rRNA gene is selected as the internal reference gene. The primers are designed as follows:
NCgl1050-F: 5'-ACACGGCACTGGGTATGTTC-3' (SEQ ID NO: 34);
NCgl1050-R: 5'-CGTTCGGATTCCTTGTAGATG-3' (SEQ ID NO: 35);
NCgl1051-F: 5'-CAGAACTTGGATTGCGTG-3' (SEQ ID NO: 36);
NCgl1051-R: 5'-GGGTGATTCCTTCAACAGC-3' (SEQ ID NO: 37).

As can be seen from FIG. 3, compared with Corynebacterium glutamicum YP097158, the expression levels of the NCgl1050 and NCgl1051 genes in the recombinant strain YPL-ΔNCgl1050-NCgl1051 are significantly decreased, confirming successful knockout of the NCgl1050 and NCgl1051 genes.

### Example 4: Construction of an engineered strain with deletion of the NCgl1706-NCgl1707 and NCgl2777 genes on the genome

Based on the Corynebacterium glutamicum ATCC13032 sequence published by NCBI, the pK18mobsacB plasmid is used to knock out the NCgl1706-NCgl1707 genes and the NCgl2777 gene in the genome of the L-lysine-producing strain YP097158 (derived from Corynebacterium glutamicum ATCC13032) (sequencing confirmed that the YP097158 strain retains intact NCgl1706-NCgl1707 genes and NCgl2777 gene on the chromosome), in order to further study the effects of these genes on L-lysine synthesis.

The correctly constructed knockout plasmid pK18-ΔNCgl2777 from Example 2 is electrotransformed into the recombinant strain YPL-ΔNCgl1706-NCgl1707 constructed in Example 1. The bacteria are cultured according to the medium composition and culture conditions shown in Table 1. Single colonies generated from the culture are identified by PCR using primers P5/P8: strains that could simultaneously amplify bands of 1480 bp and 3201 bp are positive strains, while strains that only amplified a 3201 bp band are the original strain. The positive strains are cultured on 15% sucrose solid medium (shown in Table 1) for 30 h, and then single clones are picked and screened on solid medium plates containing kanamycin and without kanamycin. Single clone strains that grow on the medium without kanamycin but do not grow on the medium containing kanamycin are further identified by PCR using primers P5/P8. Strains that could amplify a 1480 bp (SEQ ID NO: 8) band are positive strains with the coding region of the NCgl2777 gene knocked out. The gene fragment of the positive strain is amplified again using primers P5/P8 and sequenced. The strain with correct sequencing is designated YPL-ΔNCgl1706-NCgl1707-ΔNCgl2777.

The recombinant strain YPL-ΔNCgl1706-NCgl1707-ΔNCgl2777 is a recombinant Corynebacterium glutamicum obtained by replacing bases 1881670 to 1885600 of the ATCC13032 genome (GenBank: NC_03450, 22-DEC-2014) with SEQ ID NO: 5, and replacing bases 3071113 to 3074313 of the ATCC13032 genome (GenBank: NC_03450, 22-DEC-2014) with SEQ ID NO: 8, while keeping other nucleotide sequences unchanged. Specifically, the coding region of the NCgl1706 gene (nucleotide sequence: SEQ ID NO: 1, encoding the amino acid sequence shown in SEQ ID NO: 2, i.e., bases 1882467 to 1883990 of the ATCC13032 genome (GenBank: NC_03450, 22-DEC-2014)) in the genome of Corynebacterium glutamicum YP097158 is knocked out; the coding region of the NCgl1707 gene (nucleotide sequence: SEQ ID NO: 3, encoding the amino acid sequence shown in SEQ ID NO: 4, i.e., bases 1884217 to 1884888 of the ATCC13032 genome (GenBank: NC_03450, 22-DEC-2014)) is knocked out; and the coding region of the NCgl2777 gene (nucleotide sequence: SEQ ID NO: 6, encoding the amino acid sequence shown in SEQ ID NO: 7, i.e., bases 3071901 to 3073621 of the ATCC13032 genome (GenBank: NC_03450, 22-DEC-2014)) is knocked out, while other nucleotides in the genome of Corynebacterium glutamicum YP097158 are kept unchanged.

The recombinant strain YPL-ΔNCgl1706-NCgl1707-ΔNCgl2777 and Corynebacterium glutamicum YP097158 are each cultured for 24 h, and then the cells are collected by centrifugation at 4°C. RNA is extracted using an RNA extraction kit (purchased from Takara, Code No.: 9108). RNA is reverse transcribed into cDNA using a Premix-type reverse transcription kit (purchased from Takara, Code No.: RR036Q). The transcription levels of the NCgl1706, NCgl1707, and NCgl2777 genes are detected using a qPCR kit (purchased from Takara, Code No.: RR42LR). The 16S rRNA gene is selected as the internal reference gene. The primers are as referenced in Example 1 and Example 2.

As can be seen from FIG. 4, compared with Corynebacterium glutamicum YP097158, the expression levels of the NCgl1706, NCgl1707, and NCgl2777 genes in the recombinant strain YPL-ΔNCgl1706-NCgl1707-ΔNCgl2777 are significantly decreased, confirming successful knockout of the NCgl1706, NCg11707, and NCgl2777 genes.

### Example 5: Construction of an engineered strain with deletion of the NCgl1706-NCgl1707 and NCgl1050-NCgl1051 genes on the genome

Based on the Corynebacterium glutamicum ATCC13032 sequence published by NCBI, the pK18mobsacB plasmid is used to knock out the NCgl1706-NCgl1707 genes and the NCgl1050-NCgl1051 genes in the genome of the L-lysine-producing strain YP097158 (derived from Corynebacterium glutamicum ATCC13032) (sequencing confirmed that the YP097158 strain retains intact NCgl1706-NCgl1707 genes and NCgl1050-NCgl1051 genes on the chromosome), in order to further study the effects of these genes on L-lysine synthesis.

The correctly constructed knockout plasmid pK18-ΔNCgl1050-NCgl1051 from Example 3 is electrotransformed into the recombinant strain YPL-ΔNCgl1706-NCgl1707 constructed in Example 1. The bacteria are cultured according to the medium composition and culture conditions shown in Table 1. Single colonies generated from the culture are identified by PCR using primers P9/P12: strains that could simultaneously amplify bands of 1716 bp and 3391 bp are positive strains, while strains that only amplified a 3391 bp band are the original strain. The positive strains are cultured on 15% sucrose solid medium (shown in Table 1) for 30 h, and then single clones are picked and screened on solid medium plates containing kanamycin and without kanamycin. Single clone strains that grow on the medium without kanamycin but do not grow on the medium containing kanamycin are further identified by PCR using primers P9/P12. Strains that could amplify a 1716 bp (SEQ ID NO: 13) band are positive strains with the coding regions of the NCgl1050-NCgl1051 genes knocked out. The gene fragment of the positive strain is amplified again using primers P9/P12 and sequenced. The strain with correct sequencing is designated YPL-ΔNCgl1706-NCgl1707-ΔNCgl1050-NCgl1051.

The recombinant strain YPL-ΔNCgl1706-NCgl1707-ΔNCgl1050-NCgl1051 is a recombinant Corynebacterium glutamicum obtained by replacing bases 1881670 to 1885600 of the ATCC13032 genome (GenBank: NC_03450, 22-DEC-2014) with SEQ ID NO: 5, and replacing bases 1140507 to 1143897 of the ATCC13032 genome (GenBank: NC_03450, 22-DEC-2014) with SEQ ID NO: 13, while keeping other nucleotide sequences unchanged. Specifically, the coding region of the NCgl1706 gene (nucleotide sequence: SEQ ID NO: 1, encoding the amino acid sequence shown in SEQ ID NO: 2, i.e., bases 1882467 to 1883990 of the ATCC13032 genome (GenBank: NC_03450, 22-DEC-2014)) in the genome of Corynebacterium glutamicum YP097158 is knocked out; the coding region of the NCgl1707 gene (nucleotide sequence: SEQ ID NO: 3, encoding the amino acid sequence shown in SEQ ID NO: 4, i.e., bases 1884217 to 1884888 of the ATCC13032 genome (GenBank: NC_03450, 22-DEC-2014)) is knocked out; the coding region of the NCgl1050 gene (nucleotide sequence: SEQ ID NO: 9, encoding the amino acid sequence shown in SEQ ID NO: 10, i.e., bases 1141341 to 1142475 of the ATCC13032 genome (GenBank: NC_03450, 22-DEC-2014)) is knocked out; and the coding region of the NCgl1051 gene (nucleotide sequence: SEQ ID NO: 11, encoding the amino acid sequence shown in SEQ ID NO: 12, i.e., bases 1142476 to 1143015 of the ATCC13032 genome (GenBank: NC_03450, 22-DEC-2014)) is knocked out, while other nucleotides in the genome of Corynebacterium glutamicum YP097158 are kept unchanged.

The recombinant strain YPL-ΔNCgl1706-NCgl1707-ΔNCgl1050-NCgl1051 and Corynebacterium glutamicum YP097158 are each cultured for 24 h, and then the cells are collected by centrifugation at 4°C. RNA is extracted using an RNA extraction kit (purchased from Takara, Code No.: 9108). RNA is reverse transcribed into cDNA using a Premix-type reverse transcription kit (purchased from Takara, Code No.: RR036Q). The transcription levels of the NCgl1706, NCgl1707, NCgl1050, and NCgl1051 genes are detected using a qPCR kit (purchased from Takara, Code No.: RR42LR). The 16S rRNA gene is selected as the internal reference gene. The primers are as referenced in Example 1 and Example 3.

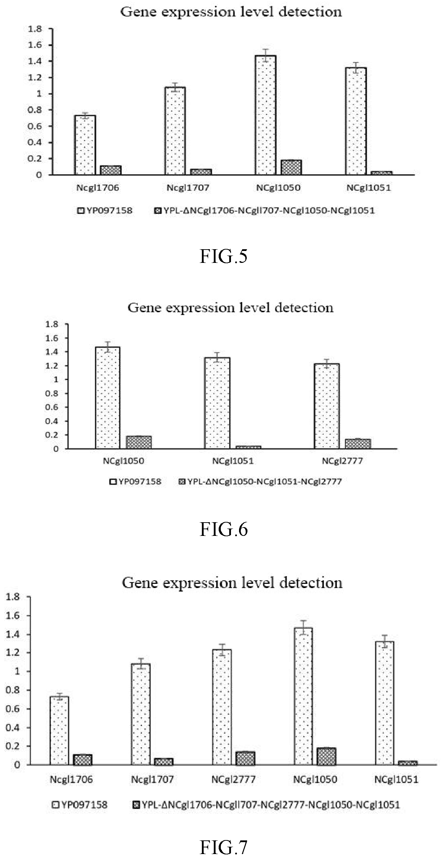

As can be seen from FIG. 5, compared with Corynebacterium glutamicum YP097158, the expression levels of the NCgl1706, NCgl1707, NCgl1050, and NCgl1051 genes in the recombinant strain YPL-ΔNCgl1706-NCgl1707-ΔNCgl1050-NCgl1051 are significantly decreased, confirming successful knockout of the NCg11706, NCgl1707, NCgl1050, and NCgl1051 genes.

### Example 6: Construction of an engineered strain with deletion of the NCgl2777 and NCgl1050-NCgl1051 genes on the genome

Based on the Corynebacterium glutamicum ATCC13032 sequence published by NCBI, the pK18mobsacB plasmid is used to knock out the NCgl2777 gene and the NCgl1050-NCgl1051 genes in the genome of the L-lysine-producing strain YP097158 (derived from Corynebacterium glutamicum ATCC13032) (sequencing confirmed that the YP097158 strain retains intact NCgl2777 gene and NCgl1050-NCgl1051 genes on the chromosome), in order to further study the effects of these genes on L-lysine synthesis.

The correctly constructed knockout plasmid pK18-ΔNCgl1050-NCgl1051 from Example 3 is electrotransformed into the recombinant strain YPL-ΔNCgl2777 constructed in Example 2. The bacteria are cultured according to the medium composition and culture conditions shown in Table 1. Single colonies generated from the culture are identified by PCR using primers P9/P12: strains that could simultaneously amplify bands of 1716 bp and 3391 bp are positive strains, while strains that only amplified a 3391 bp band are the original strain. The positive strains are cultured on 15% sucrose solid medium (shown in Table 1) for 30 h, and then single clones are picked and screened on solid medium plates containing kanamycin and without kanamycin. Single clone strains that grow on the medium without kanamycin but do not grow on the medium containing kanamycin are further identified by PCR using primers P9/P12. Strains that could amplify a 1716 bp (SEQ ID NO: 13 (1716 bp)) band are positive strains with the coding regions of the NCgl1050-NCgl1051 genes knocked out. The gene fragment of the positive strain is amplified again using primers P9/P12 and sequenced. The strain with correct sequencing is designated YPL-ΔNCgl2777-ΔNCgl1050-NCgl1051.

The recombinant strain YPL-ΔNCgl2777-ΔNCgl1050-NCgl1051 is a recombinant Corynebacterium glutamicum obtained by replacing bases 3071113 to 3074313 of the ATCC13032 genome (GenBank: NC_03450, 22-DEC-2014) with SEQ ID NO: 8, and replacing bases 1140507 to 1143897 of the ATCC13032 genome (GenBank: NC_03450, 22-DEC-2014) with SEQ ID NO: 13, while keeping other nucleotide sequences unchanged. Specifically, the coding region of the NCgl2777 gene (nucleotide sequence: SEQ ID NO: 6, encoding the amino acid sequence shown in SEQ ID NO: 7, i.e., bases 3071901 to 3073621 of the ATCC13032 genome (GenBank: NC_03450, 22-DEC-2014)) in the genome of Corynebacterium glutamicum YP097158 is knocked out; the coding region of the NCgl1050 gene (nucleotide sequence: SEQ ID NO: 9, encoding the amino acid sequence shown in SEQ ID NO: 10, i.e., bases 1141341 to 1142475 of the ATCC13032 genome (GenBank: NC_03450, 22-DEC-2014)) is knocked out; and the coding region of the NCgl1051 gene (nucleotide sequence: SEQ ID NO: 11, encoding the amino acid sequence shown in SEQ ID NO: 12, i.e., bases 1142476 to 1143015 of the ATCC13032 genome (GenBank: NC_03450, 22-DEC-2014)) is knocked out, while other nucleotides in the genome of Corynebacterium glutamicum YP097158 are kept unchanged.

The recombinant strain YPL-ΔNCgl2777-ΔNCgl1050-NCgl1051 and Corynebacterium glutamicum YP097158 are each cultured for 24 h, and then the cells are collected by centrifugation at 4°C. RNA is extracted using an RNA extraction kit (purchased from Takara, Code No.: 9108). RNA is reverse transcribed into cDNA using a Premix-type reverse transcription kit (purchased from Takara, Code No.: RR036Q). The transcription levels of the NCgl2777, NCgl1050, and NCgl1051 genes are detected using a qPCR kit (purchased from Takara, Code No.: RR42LR). The 16S rRNA gene is selected as the internal reference gene. The primers are as referenced in Example 2 and Example 3.

As can be seen from FIG. 6, compared with Corynebacterium glutamicum YP097158, the expression levels of the NCgl2777, NCgl1050, and NCgl1051 genes in the recombinant strain YPL-ΔNCgl2777-ΔNCgl1050-NCgl1051 are significantly decreased, confirming successful knockout of the NCgl2777, NCgl1050, and NCgl1051 genes.

### Example 7: Construction of an engineered strain with deletion of the NCgl1706-NCgl1707, NCgl2777, and NCgl1050-NCgl1051 genes on the genome

Based on the Corynebacterium glutamicum ATCC13032 sequence published by NCBI, the pK18mobsacB plasmid is used to knock out the NCgl1706-NCgl1707 genes, the NCgl2777 gene, and the NCgl1050-NCgl1051 genes in the genome of the L-lysine-producing strain YP097158 (derived from Corynebacterium glutamicum ATCC13032) (sequencing confirmed that the YP097158 strain retains intact NCgl1706-NCgl1707 genes, NCgl2777 gene, and NCgl1050-NCgl1051 genes on the chromosome), in order to further study the effects of these genes on L-lysine synthesis.

The correctly constructed knockout plasmid pK18-ΔNCgl1050-NCgl1051 from Example 3 is electrotransformed into the recombinant strain YPL-ΔNCgl1706-NCgl1707-ΔNCgl2777 constructed in Example 4. The bacteria are cultured according to the medium composition and culture conditions shown in Table 1. Single colonies generated from the culture are identified by PCR using primers P9/P12: strains that could simultaneously amplify bands of 1716 bp and 3391 bp are positive strains, while strains that only amplified a 3391 bp band are the original strain. The positive strains are cultured on 15% sucrose solid medium (shown in Table 1) for 30 h, and then single clones are picked and screened on solid medium plates containing kanamycin and without kanamycin. Single clone strains that grow on the medium without kanamycin but do not grow on the medium containing kanamycin are further identified by PCR using primers P9/P12. Strains that could amplify a 1716 bp (SEQ ID NO: 13) band are positive strains with the coding regions of the NCgl1050-NCgl1051 genes knocked out. The gene fragment of the positive strain is amplified again using primers P9/P12 and sequenced. The strain with correct sequencing is designated YPL-ΔNCgl1706-NCgl1707-ΔNCg12777-ΔNCgl1050-NCgl1051.

The recombinant strain YPL-ΔNCgl1706-NCgl1707-ΔNCgl2777-ΔNCgl1050-NCgl1051 is a recombinant Corynebacterium glutamicum obtained by replacing bases 1881670 to 1885600 of the ATCC13032 genome (GenBank: NC_03450, 22-DEC-2014) with SEQ ID NO: 5, replacing bases 3071113 to 3074313 of the ATCC13032 genome (GenBank: NC_03450, 22-DEC-2014) with SEQ ID NO: 8, and replacing bases 1140507 to 1143897 of the ATCC13032 genome (GenBank: NC_03450, 22-DEC-2014) with SEQ ID NO: 13, while keeping other nucleotide sequences unchanged. Specifically, the coding region of the NCgl1706 gene (nucleotide sequence: SEQ ID NO: 1, encoding the amino acid sequence shown in SEQ ID NO: 2, i.e., bases 1882467 to 1883990 of the ATCC13032 genome (GenBank: NC_03450, 22-DEC-2014)) in the genome of Corynebacterium glutamicum YP097158 is knocked out; the coding region of the NCgl1707 gene (nucleotide sequence: SEQ ID NO: 3, encoding the amino acid sequence shown in SEQ ID NO: 4, i.e., bases 1884217 to 1884888 of the ATCC13032 genome (GenBank: NC_03450, 22-DEC-2014)) is knocked out; the coding region of the NCgl2777 gene (nucleotide sequence: SEQ ID NO: 6, encoding the amino acid sequence shown in SEQ ID NO: 7, i.e., bases 3071901 to 3073621 of the ATCC13032 genome (GenBank: NC_03450, 22-DEC-2014)) is knocked out; the coding region of the NCgl1050 gene (nucleotide sequence: SEQ ID NO: 9, encoding the amino acid sequence shown in SEQ ID NO: 10, i.e., bases 1141341 to 1142475 of the ATCC13032 genome (GenBank: NC_03450, 22-DEC-2014)) is knocked out; and the coding region of the NCgl1051 gene (nucleotide sequence: SEQ ID NO: 11, encoding the amino acid sequence shown in SEQ ID NO: 12, i.e., bases 1142476 to 1143015 of the ATCC13032 genome (GenBank: NC_03450, 22-DEC-2014)) is knocked out, while other nucleotides in the genome of Corynebacterium glutamicum YP097158 are kept unchanged.

The recombinant strain YPL-ΔNCgl1706-NCgl1707-ΔNCgl2777-ΔNCgl1050-NCgl1051 and Corynebacterium glutamicum YP097158 are each cultured for 24 h, and then the cells are collected by centrifugation at 4°C. RNA is extracted using an RNA extraction kit (purchased from Takara, Code No.: 9108). RNA is reverse transcribed into cDNA using a Premix-type reverse transcription kit (purchased from Takara, Code No.: RR036Q). The transcription levels of the NCgl1706, NCgl1707, NCgl2777, NCgl1050, and NCgl1051 genes are detected using a qPCR kit (purchased from Takara, Code No.: RR42LR). The 16S rRNA gene is selected as the internal reference gene. The primers are as referenced in Examples 1-3.

As can be seen from FIG. 7, compared with Corynebacterium glutamicum YP097158, the expression levels of the NCgl1706, NCgl1707, NCgl2777, NCgl1050, and NCgl1051 genes in the recombinant strain YPL-ΔNCgl1706-NCgl1707-ΔNCgl2777-ΔNCgl1050-NCgl1051 are significantly decreased, confirming successful knockout of the NCgl1706, NCgl1707, NCgl2777, NCgl1050, and NCgl1051 genes.

### Example 8: L-Lysine Fermentation Experiment

The strains constructed in Examples 1-7 and the L-lysine-producing strain YP097158 are subjected to fermentation experiments in a BLBIO-5GC-4-H model fermenter (Shanghai Bailun Biotechnology Co., Ltd.) using the medium shown in Table 2 and the control process shown in Table 3. After fermentation, the L-lysine yield is determined by the ninhydrin colorimetric method. Each strain is tested in triplicate, and the results are shown in Table 4.

**Table 2: Fermentation Medium Formulation (the remainder is water)**

| Component | Formulation | Component | Formulation |
|---|---|---|---|
| Starch hydrolyzate | 30g/L | Ferrous sulfate | 120mg/L |
| Ammonium sulfate | 12g/L | Manganese sulfate | 120mg/L |
| Magnesium sulfate | 0.87g/L | Nicotinamide | 42mg/L |
| Molasses | 20g/L | Calcium pantothenate | 6.3mg/L |
| Acidified corn steep liquor | 3mL/L | Vitamin B1 | 6.3mg/L |
| Phosphoric acid | 0.4mL/L | Copper/zinc salt solution | 0.6g/L |
| Potassium chloride | 0.53g/L | Biotin | 0.88mg/L |
| Antifoam agent (2% Polyd imethylsiloxane) | 4mL/L | / | |

**Table 3: Fermentation Control Process**

| | | | |
|---|---|---|---|
| Calibrate DO 100% | Temperature 37°C, air flow 4 L/min, agitation 1000 rpm, tank pressure 0 MPa; calibrate after 5 min | | |
| Inoculum size | 10% | Culture temperature°C | 37°C |
| pH | pH6.9±0.05 | Dissolved oxygen (DO) | 10-30% |
| Initial conditions | Temperature 37°C, pH 6.9, tank pressure 0 MPa, air flow 3 L/min, agitation 550 rpm | | |
| Full-process control | Full-process control | | |
| | 1, When DO < 30%, sequentially increase agitation: 750 rpm → 800 rpm → air flow 4 L/min → 850 rpm → 950 rpm; | | |
| | 2, At 6 h of fermentation, increase tank pressure to 0.01 MP a; at 12 h, increase tank pressure: 0.02 MPa → 0.03 MPa → 0.04 MPa → 0.05 MPa. | | |
| Residual sugar control | Before 12 h: 0.1-0.2%; after 12 h: control residual sugar at 0.1-0.05% in combination with DO requirements | | |
| Ammonia nitrogen co ntrol | Before 12 h: 0.1-0.15; 12-32 h: 0.15-0.25; after 32 h: 0.1-0.1 5 | | |
| Fed-batch materials | 25% ammonia water, 70% concentrated sugar, 50% ammoniu m sulfate, 10% antifoam agent | | |
| Fermentation period | 48h | | |

| | | | |
|---|---|---|---|
| Note: "F12h" in the table denotes 12 hours of fermentation, "F12-F32h" denotes 12-32 hours of fermentation, and "F32h" denotes 32 hours of fermentation. | | | |

**Table 4: L-Lysine Yield and Significance Analysis**

| Corynebacterium glutamicum S train | L-Lysine Concentration (g/100mL) | | | Mean (g/100 mL) | Significance Analysis |
|---|---|---|---|---|---|
| | Ferme ntation 1 | Ferme ntation 2 | Ferme ntation 3 | | |
| YP097158 | 18.67 | 19.13 | 18.94 | 18.91 | |
| YPL-ΔNCgl1706-NCgl1707 | 19.75 | 20.06 | 19.41 | 19.74 | P<0.05 |
| YPL-ΔNCgl2777 | 19.68 | 20.14 | 20.47 | 20.10 | P<0.05 |
| YPL-ΔNCgl1050-NCgl1051 | 20.83 | 21.27 | 21.49 | 21.20 | P<0.01 |
| YPL-ΔNCgl1706-NCgl1707-ΔN Cgl2777 | 20.61 | 19.97 | 20.75 | 20.44 | P<0.05 |
| YPL-ΔNCgl1706-NCgl1707-ΔN Cgl1050-NCgll051 | 21.38 | 20.92 | 21.57 | 21.29 | P<0.01 |
| YPL-ΔNCgl2777-ΔNCgl1050-NCgl1051 | 21.65 | 21.12 | 21.73 | 21.5 | P<0.01 |
| YPL-ΔNCgl1706-NCg11707-ΔN Cgl2777-ΔNCgl1050-NCgll051 | 21.83 | 22.18 | 21.46 | 21.82 | P<0.01 |

As shown in Table 4, knockout of the coding region of the NCgl1706-NCgl1707 genes, or knockout of the coding region of the NCgl2777 gene, or knockout of the coding region of the NCgl1050-NCgl1051 genes in Corynebacterium glutamicum all contributed to an increase in L-lysine yield.

The present application has been described in detail above. For those skilled in the art, without departing from the spirit and scope of the present application and without the need for unnecessary experiments, the present application can be practiced within a broader range under equivalent parameters, concentrations, and conditions. Although specific examples have been provided in the present application, it should be understood that further improvements can be made to the present application. In summary, in accordance with the principles of the present application, the present application is intended to encompass any modifications, uses, or improvements to the present application, including changes made using conventional techniques known in the art that depart from the scope already disclosed in the present application.

### Cross-Reference to Related Applications:

The present application claims the priority of the Chinese patent application filed with the China National Intellectual Property Administration on November 30, 2023, with application number 202311620424.3 and entitled "Use of Cell Senescence-Related Proteins in the Preparation of L-Lysine", the entire content of which is hereby incorporated by reference.

### Industrial Applicability:

The present application can significantly increase the yield of L-lysine.

## Claims

1. A use of a protein, or a substance regulating expression of a gene encoding the protein, or a substance regulating activity or content of the protein, for preparation of L-lysine or improving L-lysine production, wherein the protein is any one of the following:
G1) comprising a protein encoded by an NCgl1706 gene and a protein encoded by an ncgl1707 gene;
G2) comprising a protein encoded by an NCgl2777 gene;
G3) comprising a protein encoded by an ncgl1050 gene and a protein encoded by an ncgl1051 gene;
G4) comprising a protein encoded by an NCgl1706 gene, a protein encoded by an ncgl1707 gene and a protein encoded by an NCgl2777 gene; and
G5) comprising any two or more combinations of G1), G2), G3) or G4);
Wherein, A1) the protein encoded by the ncgl1050 gene comprises any one of the following:
A1-1) a protein having an amino acid sequence of SEQ ID No.11;
A1-2) a protein having a same function as the protein encoded by the ncgl1050 gene derived from A1-1) or having more than 80% identity with the protein shown in A1-1) obtained by substitution and/or deletion and/or addition of amino acid residues in the amino acid sequence shown in SEQ ID No.11;
A1-3) a fusion protein having a same function obtained by fusing a tag to N-terminus and/or C-terminus of A1-1) or A1-2);
A2) the protein encoded by the ncgl1051 gene comprises any one of the following:
A2-1) a protein having an amino acid sequence of SEQ ID No.12;
A2-2) a protein having a same function as the protein encoded by the ncgl1051 gene derived from A2-1) or having more than 80% identity with the protein shown in A2-1) obtained by substitution and/or deletion and/or addition of amino acid residues in the amino acid sequence shown in SEQ ID No.12;
A2-3) a fusion protein having a same function obtained by fusing a tag to N-terminus and/or C-terminus of A2-1) or A2-2);
A3) the protein encoded by the NCgl2777 gene comprises any one of the following:
A3-1) a protein having an amino acid sequence of SEQ ID No.7;
A3-2) a protein having a same function as the protein encoded by the NCgl2777 gene derived from A3-1) or having more than 80% identity with the protein shown in A3-1) obtained by substitution and/or deletion and/or addition of amino acid residues in the amino acid sequence shown in SEQ ID No.7;
A3-3) a fusion protein having a same function obtained by fusing a tag to N-terminus and/or C-terminus of a3-1) or a3-2);
A4) the protein encoded by the NCgl1706 gene comprises any one of the following:
A4-1) a protein having an amino acid sequence of SEQ ID No.2;
A4-2) a protein having a same function as the protein encoded by the NCgl1706 gene derived from A4-1) or having more than 80% identity with the protein shown in A4-1) obtained by substitution and/or deletion and/or addition of amino acid residues in the amino acid sequence shown in SEQ ID No.2;
A4-3) a fusion protein having a same function obtained by fusing a tag to N-terminus and/or C-terminus of A4-1) or A4-2);
A5) the protein encoded by the ncgl1707 gene comprises any one of the following:
A5-1) a protein having an amino acid sequence of SEQ ID No.4;
A5-2) a protein having a same function as the protein encoded by the ncgl1707 gene derived from A5-1) or having more than 80% identity with the protein shown in A5-1) obtained by substitution and/or deletion and/or addition of amino acid residues in the amino acid sequence shown in SEQ ID No.4;
A5-3) a fusion protein having a same function obtained by fusing a tag to N-terminus and/or C-terminus of A5-1) or A5-2).

2. The use according to claim 1, wherein, the G5) comprises any one of the following:
P1) comprising a protein encoded by an NCgl1706 gene, a protein encoded by an ncgl1707 gene, a protein encoded by an ncgl1050 gene and a protein encoded by an ncgl1051 gene;
P2) comprising a protein encoded by an ncgl1050 gene, a protein encoded by an ncgl1051 gene, a protein encoded by an NCgl2777 gene, a protein encoded by an NCgl1706 gene and a protein encoded by an ncgl1707 gene;
P3) comprising a protein encoded by an ncgl1050 gene, a protein encoded by an ncgl1051 gene and a protein encoded by an NCgl2777 gene.

3. The use according to any one of claims 1-2, wherein the regulation is inhibition or reduction or down-regulation of expression of the gene encoding the protein or activity or content of the protein according to claim 1.

4. The use according to any one of claims 1-3, wherein the substance comprises a reagent for knocking out the gene encoding the protein.

5. The use according to claim 4, wherein the reagent may be any one of the following:
B1) a nucleic acid molecule that inhibits or reduces or down-regulates expression of the gene encoding the protein or a nucleic acid molecule that inhibits or reduces or down-regulates activity or content of the protein, wherein the nucleic acid molecule is a DNA molecule or an rna molecule;
B2) a gene encoding the rna molecule of B1);
B3) an expression cassette containing the gene of B2);
B4) a recombinant vector containing the gene of B2) or a recombinant vector containing the expression cassette of B3); and
B5) a recombinant microorganism containing the gene of B2) or a recombinant microorganism containing the expression cassette of B3) or a recombinant microorganism containing the recombinant vector of B4).

6. The use according to claim 5, wherein the DNA molecule of B1) is a nucleic acid molecule for knocking out the gene encoding the protein in a cell by homologous recombination.

7. The use according to claim 5, wherein the nucleotide sequence of the ncgl1050 gene contains SEQ ID No.9, the nucleotide sequence of the ncgl1051 gene contains SEQ ID No.11, the nucleotide sequence of the NCgl2777 gene contains SEQ ID No.6, the nucleotide sequence of the NCgl1706 gene contains SEQ ID No.1, and the nucleotide sequence of the ncgl1707 gene contains SEQ ID No.3.

8. A recombinant bacterium, wherein the recombinant bacterium does not comprise or lacks the protein according to claim 1.

9. The recombinant bacterium according to claim 8, wherein the recombinant bacterium does not comprise or lacks the gene encoding the protein according to claim 1.

10. The recombinant bacterium according to claim 9, wherein the recombinant bacterium is obtained by knocking out or silencing the gene encoding the protein in a host bacterium, wherein the host bacterium contains the gene encoding the protein.

11. The recombinant bacterium according to claim 10, wherein the recombinant bacterium comprises a bacterium.

12. The recombinant bacterium according to claim 11, wherein the recombinant bacterium comprises Corynebacterium glutamicum.

13. A use of a biological material related to the protein according to claim 1 for preparation of L-lysine or improving L-lysine production, wherein, the biological material is any one of the following:
B1) a nucleic acid molecule that inhibits or reduces or down-regulates expression of the gene encoding the protein or a nucleic acid molecule that inhibits or reduces or down-regulates activity or content of the protein, wherein the nucleic acid molecule comprises a DNA molecule or an RNA molecule;
B2) a gene encoding the RNA molecule of B1);
B3) an expression cassette containing the gene of B2);
B4) a recombinant vector containing the gene of B2) or a recombinant vector containing the expression cassette of B3); and
B5) a recombinant microorganism containing the gene of B2) or a recombinant microorganism containing the expression cassette of B3) or a recombinant microorganism containing the recombinant vector of B4).

14. A whole-cell catalyst, wherein the whole-cell catalyst comprises the recombinant bacterium according to claim 12.

15. A whole-cell catalyst, wherein the whole-cell catalyst comprises the biological material according to claim 13.

16. A method for preparing L-lysine, comprising fermenting and preparing L-lysine by using the recombinant bacterium according to any one of claims 8-12.

17. A method for preparing L-lysine, comprising fermenting and preparing L-lysine by using the biological material according to claim 13.

18. A method for preparing L-lysine, comprising fermenting and preparing L-lysine by using the whole-cell catalyst according to claim 14.

19. A method for preparing a recombinant microorganism, wherein the method comprises the following steps: down-regulating, reducing or inhibiting expression level of a gene in a target microorganism, or/and down-regulating, reducing or inhibiting expression level or activity of a protein encoded by the gene in the target microorganism, to obtain the recombinant microorganism, wherein the gene comprises the NCgl1706 gene, the ncgll707 gene, the NCgl2777 gene, the ncgl1050 gene and/or the ncgll051 gene according to claim 6.
